# EUROPEAN PATENT SPECIFICATION

(11) **EP 3 468 455 B1**
(45) Date of publication and mention of the grant of the patent: **22.04.2020**
(21) Application number: 17813860.8
(22) Date of filing: 12.06.2017
(51) Int. Cl.: A61B 5/00, A61B 5/08, A61F 5/56, A61N 1/05, A61N 1/08, A61N 1/18, A61N 1/36

(54) **ASYMMETRIC STIMULATION OF POSTERIOR CRICOARYTENOID MUSCLES**
ASYMMETRISCHE STIMULATION DER POSTERIOREN CRICOARYTENOIDEN MUSKELN
STIMULATION ASYMÉTRIQUE DES MUSCLES CRICOARYTÉNOÏDES POSTÉRIEURS

(30) Priority: 13.06.2016 US 201662349165 P
(43) Date of publication of application: 17.04.2019
(73) Proprietor: Med-El Elektromedizinische Geraete GmbH, 6020 Innsbruck (AT)
(72) Inventor: OBERPARLEITER, Markus, 6063 Rum (AT); SEDGHAMIZ, Hooman, Houston, Texas 77005 (US)
(74) Representative: Downing, Michael Philip
(86) International application number: PCT/US2017/036964
(87) International publication number: WO 2017/218383

(56) References cited:
- US-A- 5 111 814
- US-A1- 2002 156 507
- US-A1- 2010 312 302
- US-A1- 2011 264 164

## Description

### TECHNICAL FIELD

The present invention relates to implantable respiration pacing systems.

### BACKGROUND ART

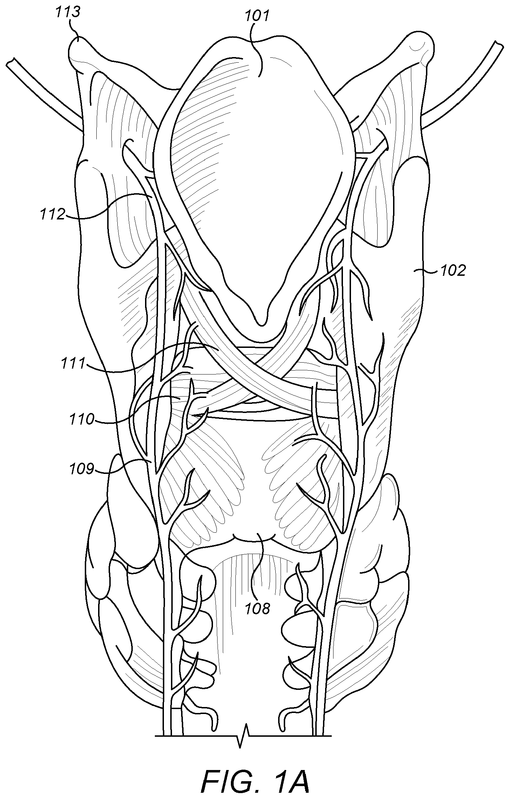

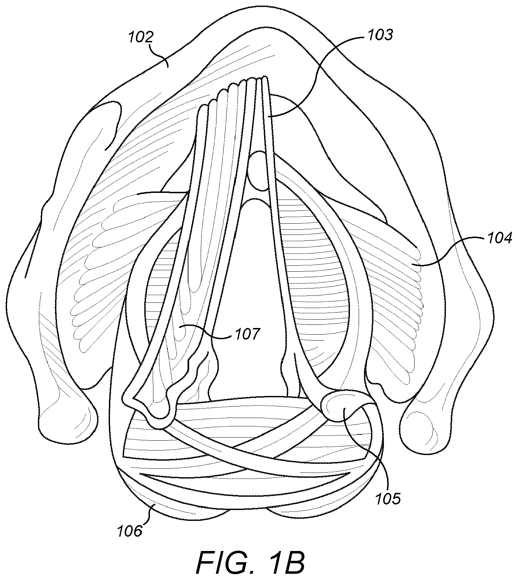

The larynx is located in the neck and is involved in breathing, producing sound (speech), and protecting the trachea from aspiration of food and water. Figure 1A shows a coronal section view and Figure 1B shows a transverse section view of the anatomy of a human larynx including the epiglottis **101,** thyroid cartilage **102,** vocal folds **103,** cricothyroid muscle **104,** arytenoid cartilage **105,** posterior cricoarytenoid muscle (PCAM) **106,** vocalis muscle **107,** cricoid cartilage **108,** recurrent laryngeal nerve (RLN) 109, transverse arytenoid muscle **110,** oblique arytenoid muscle **111,** superior laryngeal nerve **112,** and hyoid bone **113.**

The nerves and muscles of the larynx abduct (open) the vocal folds **103** during the inspiration phase of breathing to allow air to enter the lungs. And the nerves and muscles of the larynx adduct (close) the vocal folds **103** during the expiration phase of breathing to produce voiced sound. At rest, respiration frequency typically varies from 12 to 25 breaths per minute. So, for example, 20 breaths per minute result in a 3 second breath duration, with 1.5 sec inspiration, and 1.5 sec exhalation phase (assuming a 50/50 ratio). The breathing frequency changes depending on the physical activity.

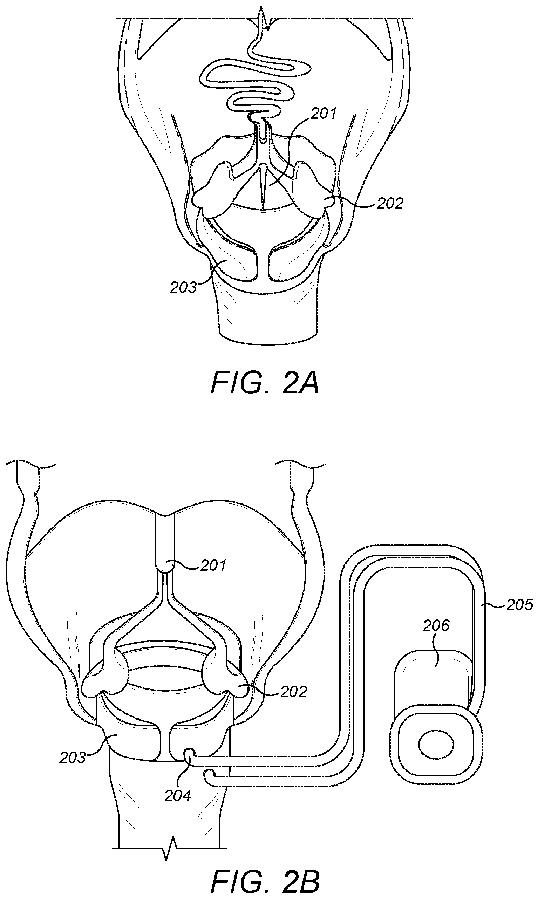

Unilateral and bilateral injuries or ruptures of the recurrent laryngeal nerve (RLN) **109** initially result in a temporal partial paralysis of the supported muscles in the larynx (and the hypolarynx). A bilateral disruption of the RLN **109** causes a loss of the abductor function of both posterior cricoarytenoid muscles (PCAM) **106** with acute asphyxia and life-threatening conditions. Figure 2A shows how the airway **201** is blocked after an injury to the RLN paralyzes the PCAM **203** and disables abduction of the vocal folds **202.** This serious situation usually requires surgical treatment of the bilateral vocal cord paralysis such as cordotomy or arytenoidectomy, which subsequently restrict the voice and puts at risk the physiologic airway protection.

Another treatment approach is to implant an electrical stimulator device to pace the PCAM. Figure 2B shows how an implanted respiration pacer **206** develops respiration pacing signals that are delivered by a pacer lead **205** to stimulation electrodes **204** that deliver electrical stimulation to the PCAM **203** during inspiration to abduct the vocal folds **202.** During expiration the vocal folds **202** relax to facilitate voicing. An implanted patient can manually adjust the stimulation frequency of the respiration pacing signals from the respiration pacer **206** by an input device on the system's external component. An assumption is, that the human body may adapt (within some locking-range) to the artificial externally applied respiration frequency.

Existing vocal fold pacer arrangements symmetrically and simultaneously stimulate both the left and right PCAM of bilateral vocal fold paralysis patients. However, the stimulation pattern is not precisely phase locked with the actual respiration effort so that some portions of the respiratory cycles may be missed.

U. S. Patent 7,069,082 discloses a bilateral vocal cord stimulation arrangement that uses a respiration sensor and provides symmetric bilateral stimulation-stimulation of both sides of the PCAM with the same stimulation pattern at the same time.

U. S. Patent 7,805,195 discloses a bilateral vocal cord stimulator for independent bilateral laryngeal muscle stimulation, where the term "independent" refers either to abduction produced only on one side of the larynx-what is also thought of as unilateral stimulation or to a spatial independently provoked abduction of the vocal folds on both sides. Furthermore, the system described uses a respiration sensor to detect respiration activity. Thus, unilateral or bilateral stimulation of the vocal folds is always realized synchronously to a certain respiration phase. Therefore, our proposed system does not require any respiration sensing feedback.

US2010312302 discloses a laryngeal stimulation system including a sensor for detecting an occurrence of a respiratory signal in a subject and a laryngeal stimulator adapted for coupling to a laryngeal muscle and operable to stimulate the laryngeal muscle in response to the sensor detecting the occurrence of the respiratory signal is provided. A method for laryngeal stimulation including detecting an occurrence of a respiratory signal in a subject and then stimulating a laryngeal muscle in response to detecting the occurrence of the respiratory signal is also discussed.

### SUMMARY

Embodiments of the present invention are directed to a respiration implant system for an implanted patient with impaired breathing that includes laryngeal stimulating electrodes configured to interface with left and right posterior cricoarytenoid muscles (PCAM) of a patient larynx to deliver respiration pacing signals to the PCAM to promote patient breathing. And a pacing processor is configured to generate the respiration pacing signals to alternatingly stimulate the left and right PCAM one at a time so as to always have one of the left and right PCAM in an unstimulated resting state.

In further specific embodiments, the pacing processor may be configured to generate the respiration pacing signals asynchronously without a respiration sensing signal. Or there may be a respiration sensor configured to develop a respiration signal representing respiration activity of the implanted patient, and the pacing processor is configured to receive the respiration sensing signal and generate the respiration pacing signals synchronously with respiration activity in the implanted patient.

The pacing processor may be configured to generate the respiration pacing signals so that the left and right PCAM are each stimulated every other breath, or to generate respiration pacing signals so that the left and right PCAM are stimulated out of phase from each other. The laryngeal stimulating electrodes may be configured to be placed on the left and right PCAM or the left and right recurrent laryngeal nerves.

### BRIEF DESCRIPTION OF THE DRAWINGS

Figure 1A shows a coronal section view and Figure 1B shows a transverse section view of the anatomy of a human larynx.
Figure 2A shows how an airway restricts when the vocal folds cannot properly abduct.
Figure 2B shows how an implanted respiration pacing system can deliver electrical stimulation signals to abduct the vocal folds.
Figure 3A compares inspiration signals and stimulation patterns for conventional symmetric pacing arrangements.
Figure 3B compares left and right side inspiration signals and stimulation patterns for asymmetric pacing arrangements according to an embodiment of the present invention.
Figure 3C compares left and right side inspiration signals and stimulation patterns for asymmetric pacing arrangements according to another embodiment of the present invention.
Figure 4 shows various stimulation parameters that may be adjusted in asymmetric stimulation channels.

### DETAILED DESCRIPTION

Various embodiments of the present invention are directed to improved respiration implants that are configured to deliver asymmetric and asynchronous stimulation of PCAM in bilaterally paralyzed patients. Compared to the existing conventional symmetric stimulation, left and right PCAM are stimulated separately with a phase and/or frequency shift. Therefore, one PCAM is at rest when the other is being stimulated. Such arrangements avoids the need of a respiration sensor as the opening of the vocal folds become independent from the respiration frequency and overcomes the lack of existing systems which have no respiration sensors implemented.

Thus, embodiments of the present invention are directed to a respiration implant system for an implanted patient with impaired breathing that includes laryngeal stimulating electrodes configured to interface with left and right posterior cricoarytenoid muscles (PCAM) of a patient larynx to deliver respiration pacing signals to the PCAM to promote patient breathing. And a pacing processor is configured to generate the respiration pacing signals to alternatingly stimulate the left and right PCAM one at a time so as to always have one of the left and right PCAM in an unstimulated resting state.

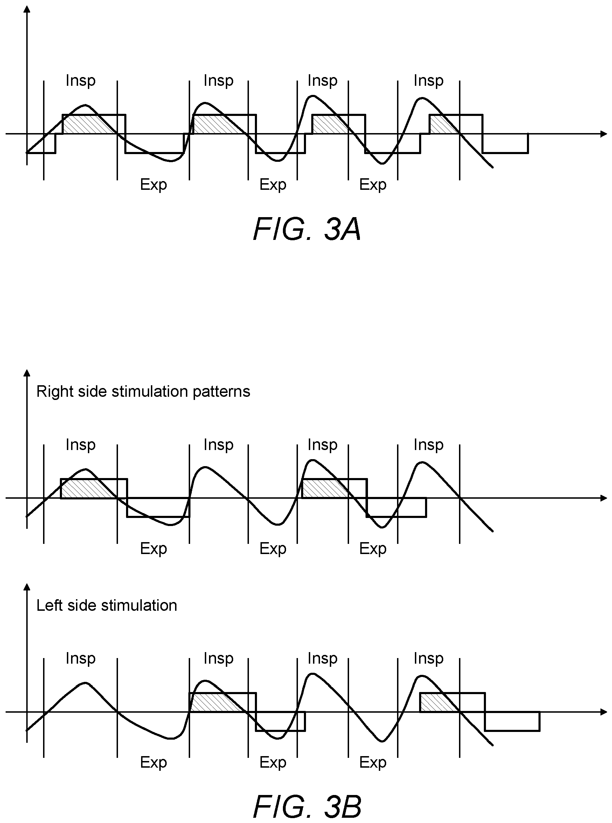

Figure 3A compares inspiration signals and stimulation patterns for conventional symmetric pacing arrangements where the natural respiratory pattern is shown by the regular sine wave signal with the positive phase representing inspiration and the negative phase representing expiration. The superimposed rectangular waveform shows the symmetric stimulation pattern that is simultaneously and in phase provided to both left and ride side PCAM. The shaded areas represent the portion of the inspiratory phase that is captured by the symmetric stimulation pattern. This stimulates both left and right side PCAM on every breath, which can produce problems of muscle fatigue from the amount of recurring electrical stimulation. Note that in Figure 3A there are portions when the actual respiratory pattern has a positive phase, but the stimulation pattern does not (unshaded white areas). This represents periods of missed inspiration when the body is straining to breath in, but the paralyzed vocal folds are not stimulated into abduction so that airway is actually by the incoming air pressing against the closed vocal folds.

Figure 3B compares left and right side inspiration signals and stimulation patterns for asymmetric pacing arrangements according to an embodiment of the present invention. The natural inspiration is represented by the same regular sine wave signal, but the asymmetric stimulation pattern as applied to each side every other breath out of phase. Thus for each breath, one side of the PCAM receives the respiration pacing signals, while the other side PCAM is left in an unstimulated resting state. The same amount of the inspiratory period is captured (i.e., there is the same amount of missed inspiration) as with conventional symmetric stimulation, but the amount of stimulation on each side is halved, reducing fatigue of the PCAM. The amount of current flow and overall power consumption are halved as well.

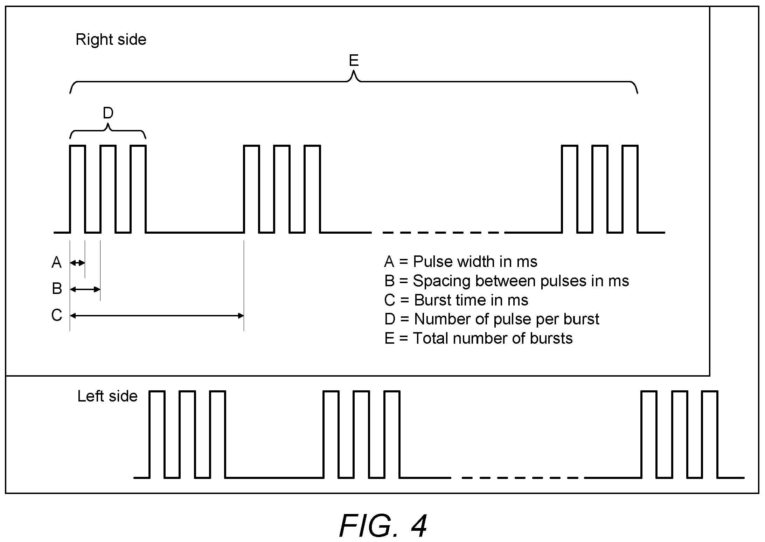

The stimulating electrodes may be placed on or in the PCAMs themselves or adjacent their respective recurrent laryngeal nerves (RLNs). In both cases, the muscles are stimulated via the innervating nerve fibers or nerve fiber ends. Various stimulation parameters as shown in Figure 4 may be adjusted such as pulse width, pulses burst time, the number of pulses per burst and the phase shift between the channels.

Providing symmetric bilateral stimulation both vocal folds are opened at the same time to provide better air flow through the vocal folds. Assume As denotes the open area between the two opened vocal cords in symmetric stimulation. And assume the case of asymmetric bilateral stimulation when only one of the two PCAMs is open at a time. So it may appear that only the half amount of air may pass through the vocal folds as compared to for symmetric stimulation and that the open area in this asymmetric case *Aas* would be *As*/*2.* But for normal daily life activities, the volume of air flow through an airway with only one opened vocal fold in fact is sufficient, indeed, the air that can pass through the airway during asymmetric stimulation is significantly more than just half of the amount of air compared to the symmetric case; so in terms of open areas: *Aas > As*/2. As a result, each PCMA has more time to relax until it has to contract again and so can contract more and open the vocal fold more. Even at significantly reduced power consumption from that with symmetric bilateral stimulation systems, the patient is supported for both daily life activities and even for elevated activities that require more oxygen. The stimulation pulse amplitudes do need to be limited so that the patient is always able to voluntarily or reflexively close the vocal folds to retain intact the natural protection mechanism against swallowing.

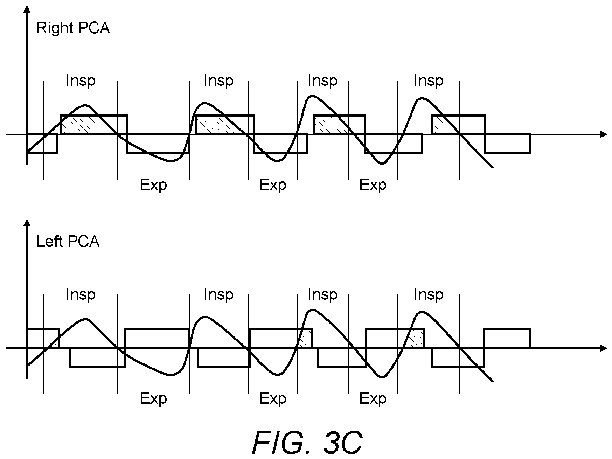

Figure 3C compares left and right side inspiration signals and stimulation patterns for asymmetric pacing arrangements according to another embodiment of the present invention where the same burst time is used for both the left and right stimulation patterns. In such an arrangement, muscle fatigue will be just as significant as in conventional symmetric bilateral stimulation, but the greater benefit is that all of the inspiratory phases are captured with a simple 180° out of phase stimulation of left and right PCAM. This means the air way is always half opened at any time. The stimulation amplitude requirement has to be considered here as well.

In further embodiments the pacing processor is configured to generate the respiration pacing signals so that left and right PCAM are in a partially open state at the same time while maintaining the alternating stimulation scheme. Partially open may be considered as being open up to 50% or less, but not 100% which would be bilateral symmetric stimulation. A stimulation scheme in which both PCAM are to a certain (small) degree in an open state at the same time ensures that pathway is permanently open. As in the previous case, the stimulation amplitude requirement has to be considered here as well.

Asymmetric pacing does not generate any adverse effect on phonation either. Compared to other rehabilitation techniques such as cordotomy or arytenoidectomy, phonation ability is unaffected. And similarly, the natural protection mechanism against swallowing also is fully preserved.

In further embodiments left and right stimulation electrodes may comprise of a plurality of electrodes, i.e. the left or the right or both of them. Providing such plurality of electrodes allows for advanced stimulation paradigms. E.g. the respiration pacing signals may be delivered to the left and/or right PCMA(s) in a way such that not all electrodes out of the plurality of electrodes are stimulated at the same time. In a specific embodiment, the electrodes out of the plurality of electrodes may be stimulated one after the other, i.e. in a carrousel stimulation, in order to mitigate (avoid or reduce) fatigue of the PCMA muscle(s) near the location of electrode placement. This way, the opening of the vocal folds can be further optimized and better adapted to the patient's needs.

Embodiments of the invention may be implemented in part in any conventional computer programming language such as VHDL, System C, Verilog, ASM, etc. Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (e.g., a diskette, CD-ROM, ROM, or fixed disk) or transmittable to a computer system, via a modem or other interface device, such as a communications adapter connected to a network over a medium. The medium may be either a tangible medium (e.g., optical or analog communications lines) or a medium implemented with wireless techniques (e.g., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g.,* shrink wrapped software), preloaded with a computer system (*e.g.,* on system ROM or fixed disk), or distributed from a server or electronic bulletin board over the network (*e.g.,* the Internet or World Wide Web). Of course, some embodiments of the invention may be implemented as a combination of both software (*e.g.,* a computer program product) and hardware. Still other embodiments of the invention are implemented as entirely hardware, or entirely software (*e.g.,* a computer program product).

## Claims

1. A respiration implant system for a patient with impaired breathing, the system comprising:
laryngeal stimulating electrodes configured to interface with left and right posterior cricoarytenoid muscles (PCAM) of a patient larynx to deliver respiration pacing signals to the PCAM to promote patient breathing; and
a pacing processor configured to generate the respiration pacing signals **characterised in that** said pacing processor is further configured to alternatingly stimulate the left and right PCAM one at a time so as to always have one of the left and right PCAM in an unstimulated resting state.

2. The system according to claim 1, wherein the pacing processor is configured to generate the respiration pacing signals asynchronously to a respiration cycle without a respiration sensing signal.

3. The system according to claim 1, wherein the pacing processor is configured to generate the respiration pacing signals so that the left and right PCAM are each stimulated every other breath.

4. The system according to claim 1, wherein the pacing processor is configured to generate respiration pacing signals so that the left and right PCAM are stimulated out of phase from each other.

5. The system according to claim 1, wherein the pacing processor is configured to generate the respiration pacing signals so that left and right PCAM are in a partially open state at the same time.

6. The system according to claim 1, wherein the laryngeal stimulating electrodes are configured to be placed on the left and right PCAM.

7. The system according to claim 1, wherein the laryngeal stimulating electrodes are configured to be placed on left and right recurrent laryngeal nerves.

8. The system according to claim 7, wherein the respiration pacing signals are delivered to the PCAM of the patients larynx in a carrousel stimulation paradigm so as to mitigate muscle fatigue near the location of electrode placement.

9. The system according to claim 1, wherein the stimulating electrodes comprise of a plurality of electrodes.

## Patentansprüche

1. Respirationsimplantatsystem für einen Patienten mit beeinträchtigter Atmung, wobei das System umfasst:
laryngeale Stimulationselektroden, die konfiguriert sind, um eine Schnittstelle zu linken und rechten posterioren cricoarytenoiden Muskeln (PCAM) des Larynx eines Patienten zu bilden, um Respirationsschrittmachersignale an die PCAM abzugeben, um das Atmen des Patienten zu fördern; und
einen Schrittmacherprozessor, der konfiguriert ist, um die Respirationsschrittmachersignale zu generieren, **dadurch gekennzeichnet, dass** der Schrittmacherprozessor des Weiteren konfiguriert ist, um alternierend den linken und den rechten PCAM einen nach dem anderen zu stimulieren, damit sich immer einer von dem linken und dem rechten PCAM in einem unstimulierten Ruhezustand befindet.

2. System nach Anspruch 1, wobei der Schrittmacherprozessor konfiguriert ist, um die Respirationsschrittmachersignale asynchron zu einem Respirationszyklus ohne ein Respirationsabfühlsignal zu generieren.

3. System nach Anspruch 1, wobei der Schrittmacherprozessor konfiguriert ist, um die Respirationsschrittmachersignale so zu generieren, dass der linke und der rechte PCAM jeweils bei jedem zweiten Atemzug stimuliert werden.

4. System nach Anspruch 1, wobei der Schrittmacherprozessor konfiguriert ist, um die Respirationsschrittmachersignale so zu generieren, dass der linke und der rechte PCAM jeweils phasenverschoben zueinander stimuliert werden.

5. System nach Anspruch 1, wobei der Schrittmacherprozessor konfiguriert ist, um die Respirationsschrittmachersignale so zu generieren, dass der linke und der rechte PCAM jeweils gleichzeitig in einem teilweise offenen Zustand vorliegen.

6. System nach Anspruch 1, wobei die laryngealen Stimulationselektroden so konfiguriert sind, dass sie auf dem linken und rechten PCAM zu platzieren sind.

7. System nach Anspruch 1, wobei die laryngealen Stimulationselektroden so konfiguriert sind, dass sie auf dem linken und rechten Rekurrenznerv zu platzieren sind.

8. System nach Anspruch 7, wobei die Respirationsschrittmachersignale in einem Karussellstimulationsparadigma an die PCAM des Larynx des Patienten abgegeben werden, um so Muskelermüdung in der Nähe der Stelle der Elektrodenplatzierung zu verringern.

9. System nach Anspruch 1, wobei die Stimulationselektroden eine Vielzahl von Elektroden umfassen.

## Revendications

1. Système d'implant respiratoire pour un patient ayant une respiration altérée, le système comprenant :
des électrodes de stimulation laryngée configurées pour s'interfacer avec les muscles cricoaryténoïdes postérieurs gauche et droit (PCAM) du larynx d'un patient pour délivrer des signaux de stimulation respiratoire aux PCAM afin de favoriser la respiration du patient ; et
un processeur de stimulation configuré pour générer les signaux de stimulation respiratoire
**caractérisé en ce que** ledit processeur de stimulation est en outre configuré pour stimuler alternativement les PCAM gauche et droit un à la fois de manière à toujours avoir l'un des PCAM gauche et droit dans un état de repos non stimulé.

2. Système selon la revendication 1, le processeur de stimulation étant configuré pour générer les signaux de stimulation respiratoire de manière asynchrone par rapport à un cycle de respiration sans signal de détection de la respiration.

3. Système selon la revendication 1, le processeur de stimulation étant configuré pour générer les signaux de stimulation respiratoire de sorte que les PCAM gauche et droite sont chacun stimulés une respiration sur deux.

4. Système selon la revendication 1, le processeur de stimulation étant configuré pour générer les signaux de stimulation respiratoire de sorte que les PCAM gauche et droite sont stimulés de manière déphasée l'un par rapport à l'autre.

5. Système selon la revendication 1, le processeur de stimulation étant configuré pour générer les signaux de stimulation respiratoire de sorte que les PCAM gauche et droit sont dans un état partiellement ouvert en même temps.

6. Système selon la revendication 1, les électrodes de stimulation laryngée étant configurées pour être placées sur les PCAM gauche et droit.

7. Système selon la revendication 1, les électrodes de stimulation laryngée étant configurées pour être placées sur les nerfs laryngés récurrents gauche et droit.

8. Système selon la revendication 7, les signaux de stimulation respiratoire étant délivrés au PCAM du larynx des patients dans un paradigme de stimulation en carrousel afin d'atténuer la fatigue musculaire près de l'emplacement de l'électrode.

9. Système selon la revendication 1, les électrodes de stimulation comprenant une pluralité d'électrodes.
